(19) **Europäisches Patentamt**

**European Patent Office**

**Office européen des brevets**

(11) **EP 1 637 163 A1**

(12) **EUROPEAN PATENT APPLICATION**

(43) Date of publication:
22.03.2006 Bulletin 2006/12

(51) Int Cl.:
*A61K 47/00* (2006.01)    *G01N 33/48* (2006.01)

(21) Application number: **04022450.3**

(22) Date of filing: **21.09.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **Kluge, Andreas**
**01326 Dresden (DE)**

(72) Inventors:
• **Karger, Norbert**
**01309 Dresden (DE)**
• **Kluge, Andreas**
**01326 Dresden (DE)**

(74) Representative: **Vossius & Partner**
**Siebertstrasse 4**
**81675 München (DE)**

(54) **Indicator of therapeutic compliance**

(57)    The present invention provides a composition comprising an agent for the prevention, alleviation, treatment or diagnosis of a disease and a pharmaceutically acceptable excipinet, wherein said excipient is enriched with at least one type of a stable rare isotopes as a indicator of compliance with an administration plan. Furthermore, the invention provides a methods for determination of the compliance with an administration plan for a composition and uses of compositions of the invention.

EP 1 637 163 A1

**Description**

The present invention relates to a composition comprising an agent for the prevention, alleviation, treatment or diagnosis of a disease and a pharmaceutically acceptable excipient, wherein said excipient is enriched with at least one type of a stable rare isotopes as a quantitative indicator of compliance with an administration plan. Furthermore, the invention relates to a method for determination of the compliance with an administration plan for a composition and uses of compositions according to the invention.

Several documents are cited throughout the text of this specification. The disclosure content of each of the documents cited herein (including any manufacturer's specifications, instructions, etc.) is herewith incorporated by reference.

Therapeutic compliance is a quantitative measure of the actual drug intake or administration (e.g. ingestion) by a patient or volunteer, relative to the administration plan (the prescribed dosing regimen). Lack of compliance with medication regimens is common in clinical practice (Del Boca et al. 1996, Urquhart 1994, Ellard et al. 1980). This also applies to drug trials with healthy volunteers or patients.

The degree of compliance varies with a number of factors, including the illness being treated, complexity of dosing regimen, extent of adverse side effects, and duration of treatment.

In order to be able to properly judge the effectiveness (or the lack thereof) of a given treatment, it is essential to know whether the patient (in a treatment setting), or the volunteer (in the case of a clinical trial) has followed the prescribed intake regimen of medication. Since the statements of the subjects cannot always be trusted, a method is needed to objectively measure the degree of compliance with the treatment regimen. This information is needed to quantify the efficacy of a newly developed drug in clinical trials, and will help the physician choose the best treatment for a given patient (i.e., switch to another form of treatment if one type is proven to be ineffective). Failure to ascertain the extent of noncompliance in a pharmacotherapy trial may lead to invalid conclusions, e.g., that the medication lacked efficiency (because of poor compliance among subjects receiving active medication).

The problem of patient noncompliance is well documented in the literature (Greenberg 1984, Haynes et al. 1979). Several methods have been suggested to overcome this problem (Urquhart 1994):

- Patient interviews, diaries, and counts of returned, untaken doses: these methods suffer from subjectivity and unreliability. They commonly overestimate compliance.
- Electronic devices (drug containers with electronic devices in the lid which record the times of opening of container): opening of the lid does not prove ingestion of the drug. Also, the bulkiness of the containers may confound compliance measurements for medication regimens that involve divided doses, in that efforts to avoid carrying the container out of the home may result in multiple doses of the drug being removed at once.

- Direct measurement of drug concentration: the detection of the drug being studied, or its metabolites, in biological fluids, is generally expensive and requires highly specialised equipment. Due to the generally low halflife of the drug in the body, this method can only detect compliance over the last few days before the measurement.
- Indicator substances: Low-dose, slow turn-over chemical indicators have been tested and used as compliance indicators. The method involves mixing a small amount of these substances (e. g., riboflavin, digoxine, isoniacid, phenobarbital, ofloxazine, etc.) with the medication to be taken, and analysing body fluids (blood, urine) to detect these substances. A positive result will prove ingestion of the drug, but this technique also has a number of limitations/disadvantages:

  o It might prove dose ingestion during a few days before the examination only
  o Quantification of the amount of ingested medication is difficult
  o In the case of riboflavin (= vitamin B2), the method is biased by varying amounts of intake with the food
  o Other substances are pharmacologically active, and might even be toxic in higher concentrations — both highly unwanted properties (e.g., Isoniacid)

In the state of the art uses of substances labeled with stable (as opposed to non-stable radioactive) isotopes are well established tools for research of physiologic parameters in medicine and biology (e. g., Avogaro et al. 2003, Krumbiegel et al. 1988, Romijn et al. 2000, Wutzke et al. 1985). When used in humans, the aim generally is to study various metabolic pathways (Sidossis et al. 1995, Wolfe et al. 1984, Woerle et al. 2002, Korach-Andre et al. 2002). Furthermore, the use of stable $^2$H-isotopes, as well as $^{13}$C-istotopes in the functional gastrointestinal diagnosis of intestinal diseases by breath tests has been reported (Lembcke 1997, Romagnuolo J. 2002).

In view of the unsatisfactory means for determining compliance, the technical problem underlying the present invention was to provide means and methods to determine and to monitor the compliance of the actual administration of a composition relative to the administration plan (the prescription).

The solution to said technical problem is achieved by providing the embodiments characterized in the claims.

Accordingly, the present invention provides a composition comprising an agent for the prevention, alleviation, treatment or diagnosis of a disease and a pharmaceutically acceptable excipient, wherein said excipient is enriched with at least one type of a stable rare isotopes as a indicator of compliance with an administration plan for the composition.

In accordance with this invention, the term "composition" relates to a composition for administration to a subject, preferably a human subject. Said subject may be either a healthy or a diseased volunteer in a drug trial or a patient on a therapeutic regimen. The definition of the term explicitly comprises "pharmaceutical compositions" as well as "diagnostic compositions". A pharmaceutical composition in line with the present invention comprises as an active ingredient at least one agent for the prevention, alleviation or treatment of a disease. In contrast, a diagnostic composition according to the invention does not necessarily comprise such an agent for the prevention, alleviation or treatment of a disease but means for the diagnosis of such disease.

Preferably, the composition is for oral, rectal, vaginal, parenteral, topical or transdermal administration. These compositions can be administered to the subject at a suitable dose. The dosage regimen will be determined by the attending physician and clinical factors. As it is well known in the medical arts, dosages for any one patient depends upon many factors, including the patient's size, body surface area, age, the particular compound to be administered, sex, time and route of administration, general health, and other drugs being administered concurrently.

The bases for the formulation of rectally or vaginally administered compositions such as suppositories and vaginal ovules comprise lipid-containing and watersoluble preparations, with cocoa butter, hard fat, Macrogol 6000, PEG 6000 or mixtures of macrogoles or glycerine gelatine.

For the formulation of dosage forms for oral application particularly aerosol, sucrose, starch, particularly potato starch and mixtures of at least two of the above-mentioned substances are used as carrier/vehicle.

The composition of the present invention further comprises a pharmaceutically acceptable excipient. Examples of suitable pharmaceutical excipients are well known in the art.

Excipients used according to the invention comprise carriers, additives and dilutens such as e.g. capsules, vehicles, conservants, colourants, disintegrating agents, binders, emulsifiers, solubilisers, wetting agents, solvents, buffering agents, gel-forming agents, thickeners, film-forming agents, lubricants, glidants, form-separating agents, flow-regulating agents, sorbents and additives such as antioxidants, taste- and smell-correcting agents. Carriers and diluents for liquidly administered compositions include phosphate buffered saline solutions, water, emulsions, such as oil/water emulsions, various types of wetting agents, sterile solutions, etc. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Compositions comprising such carriers can be formulated by well known conventional methods.

Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like.

The term "stable rare isotopes" in the context of the present invention defines an naturally occurring isotope which is stable (non-radioactive) and having a natural abundance of less than 10 percent relative to the total abundance of the given element. Examples for stable rare isotopes comprise $^2H$, $^{13}C$, $^{15}N$, $^{17}O$ and $^{18}O$.

It has been surprisingly found in accordance with the present invention that stable rare isotopes may be used as indicators for compliance of a composition. The use of labelled substances described herein differs significantly in two general aspects compared with the above described state of the art:

(i) it is neither necessary nor intended to study a specific metabolic pathway, but rather to find a summation signal over all involved metabolic pathways.
(ii) the measurement signal is the result of a long-term distribution of the stable isotope in the various body compartments, as opposed to the generally short-term analysis used for existing studies.

The present inventive concept is to use a indicator technique involving substances enriched with stable (as opposed to non-stable radioactive), rare isotopes as a measure of therapeutic compliance. Said substances enriched with the stable rare isotopes may be readily metabolized in the body. However, it is also envisaged that substances are enriched with the stable rare isotopes, which are not or only in low quantities metabolized in the body of the subject, the compliance of which is to be analyzed (essential substances). For the determination of the compliance of the subject with an administration plan for a composition, the relative amount of the respective isotope in body fluids or tissues is analyzed. For example, the stable rare isotopes $^{15}N$ and $^{13}C$ behave physiologically almost like their more commonly occurring counterparts (e.g., $^{14}N$, $^{12}C$), and are metabolised in the body depending on the molecule they are part of. The isotope composition of body fluids and tissue reflects the added intake of these isotopes. After measuring of suitable calibration curves, it is possible to quantify the intake of the labelled substances. For the quantification of compliance, these substances may be mixed with the medication to be taken, and the measurement thereby also allows the calculation of compliance.

The method of the invention is defined in more detail herein below and exemplified in some embodiments in the appended examples.

According to a preferred embodiment of the invention the at least one pharmaceutically acceptable excipient (carrier, additive or diluent) comprised in the composition is selected from the group of saccharides, gelatin, creatine, amino acids, (poly)peptides, proteins, lipids, fatty acids, and inorganic or organic salts.

In connection with the present invention, the term "saccarides" defines different kinds of sugars which are used in the formulation of pharmaceutical and diagnostic compositions. Particularly compromised by said definition are monosaccarides, oligosaccarides, polysaccarides as well as saccarides in macromolecular forms such as amylose and starch.

The term "(poly)peptide" as used herein describes a group of molecules which comprise the group of peptides, as well as the group of polypeptides. The group of peptides consists of molecules with up to 30 amino acids, the group of polypeptides consists of molecules with more than 30 amino acids.

According to the invention the term "fatty acids" comprises saturated (aliphatic carbonic acids such as e.g. acetic acid, butyric acid, palmitic acid or stearic acid), as well as unsaturated fatty acids (comprising singular unsaturated, double unsaturated and further unsaturated fatty acids such as e.g. oleic acid, linoleic acid, arachidonic acid) and branched as well as non-branched fatty acids. Particularly envisaged is the use of essential fatty acids.

The composition of the invention in further preferred embodiments comprises pharmaceutically acceptable excipients (carriers, additives and/or diluents), wherein the at least one type of stable rare isotope is a $^{13}C$ or $^{15}N$ isotope.

In line with the invention the amino acid(s) or salt(s) thereof may be enriched with at least one type of stable rare isotopes. In a preferred embodiment the stable rare isotope enriched in the amino acid(s) or salt(s) thereof is a $^{15}N$ isotope.

It is particularly preferred, that the at least one amino acid is selected from the group of leucine, histidine, arginine, isoleucine, lysine, methionine, phenylalanine, threonine, tryptophan, valine, glycin, alanin, asparagines, glutamine, cystein and serine.

The specific characteristic of the use of essential amino acids such as leucine, histidine, arginine, isoleucine, lysine, methionine, phenylalanine, threonine, tryptophan or valine is the fact the due to their essentiality such amino acids are metabolized only in reduced amounts compared to non-essential amino acids. Thus, the use of such essential amino acids the mean residence time of the stable rare isotopes in the body of the subject which is tested for the compliance of the actual drug administration relative to the administration plan. An increase or prolongation of the mean residence time of the stable rare isotopes in the body of the subject allows an extension of the interval in which the compliance is monitored. Essential amino acids can be expected as a group to be metabolised to a lesser extent, when compared to non-essential amino acids, hence conferring a longer-lasting "duration" of the indicator signal (i.e. the half life).

However, the question of metabolism of the labeled substance is not decisive for the means and methods of the present invention.

The particular superior feature of amino acids such as glycin, alanin, asparagines, glutamine, cysteine, arginine or serine is the relative high content of N in the percentage of molecular weight. Thus, by using amino acids of the latter group the intake of stable rare isotopes relative to the weight of the administered composition can be increased. A high molar content of $^{15}N$ in the amino acid chosen as an indicator, allows to minimise the required absolute quantity of the indicator amino acid to be added to the pharmaceutical composition.

Experiments in which some of the particularly preferred amino acids were used, are described in the appended examples.

According to an alternatively particularly preferred embodiment of the composition of the present invention the saccaride is glucose, fructose, lactose, saccharose, hypromellose and/or starch. In the preferred embodiment that the stable rare isotope enriched in the saccaride is a $^{13}C$ isotope the advantage is that saccarides are characterized by a the relative high content of C in the percentage of molecular weight. Thus, by using saccarides the intake of stable rare $^{13}C$ isotopes relative to the weight of the administered composition can be increased.

A further embodiment of the present invention relates to an in vitro or ex vivo method for determination of the compliance with an administration plan for a composition. Said method comprises the step of determining the amount of at least one type of a stable rare isotope in a sample, wherein said at least one type of stable rare isotope was enriched in a pharmaceutically acceptable excipient (carrier, additive, capsule or diluent) comprised in said composition.

The composition may be a composition as described herein above.

The administration plan for a composition may comprise the regular administration of said composition over a period of at least a week. However, the regular administration of said composition over a period of at least 12 month is also envisaged by the present invention. Thus, the period may be more than one year, about or exactly twelve months, nine months, six months, sixteen weeks, twelve weeks, ten weeks, eight weeks, six weeks, four weeks, two weeks or at least one week and any suitable time frame in between. It is particularly preferred to investigate intervals, which are typical visit intervals in clinical trials, or in routine medical practise, such as weekly, bi-weekly, monthly, 6-weekly, quaterly.

The term "sample" denotes in the context of the present invention body sample, such as samples of body fluids, excrements as well as samples of tissues of a subject/ patient. According to the present invention the amount of at least one type of a stable rare isotope may also be determined in a breath sample. Preferable the subject/ patient the sample is taken from is human.

Tissue samples explicitly comprise in the context of the present invention samples of hair (with or without the root of the hair), samples from nails or other dermal material, such as epidemal detritus, mucosal swabs, such as, but not limited to oral mucosal, tonsillar, rectal, genital or nasal swabs, as well as samples obtained by surgical techniques including minimal invasive techniques such as biopsy as well as more invasive techniques.

Furthermore, the present invention relates to a method for determination of the compliance with an administration plan for a composition, the method comprising the step of

(a) administering to a subject/patient a composition comprising an agent for the prevention, alleviation, treatment or diagnosis of a disease and a pharmaceutically acceptable excipient (carrier, additive, capsule or diluent) enriched with at least one type of a stable rare isotope as a indicator for compliance;
(b) taking a sample from said subject/patient; and
(c) determining the amount of said stable rare isotope in the sample.

The interval between two administrations of the composition will depend on the particular characteristics of the compound. Said characteristics comprise e.g. the effectiveness of a pharmaceutical composition, the desired level or the mean residence time of the compound in the body or body compartment of the subject. The intervals between the administrations may also differ during the general administration. Thus, for example the interval between a first and a second administration may be the same, longer or shorter than the interval between the second and a third administration. The same holds true for further intervals of administration.

According to preferred embodiments of the methods of the invention said samples are samples of a body fluid. More preferably, said body fluid is blood, serum, saliva, urine or sweat.

The preparation of the sample prior to the step of determining the amount of said stable rare isotope in the sample may comprise that the sample is dried, milled and/or burned. Corresponding steps for the preparation of the sample are described in more detail in the appended examples.

According to a further preferred embodiment of the invention, the amount of the stable rare isotope in the sample is determined by mass spectrometry. The use of mass spectrometry for the determination of the amount of the stable rare isotope in a sample is known to the person skilled in the art and represents a standard procedure, see e.g. De Hoffmann and Stroobant (Mass Spectrometry: Principles and Applications).

Preferably the amount of the stable rare isotope is determined in a series of samples taken from a subject/ patient.

A preferred interval between the collection of the samples is about or exactly twelve months. However, the interval between the collection of the samples may also exceed twelve months. Also alternatively preferred are intervals about or exactly nine months, six months, sixteen weeks, twelve weeks, ten weeks, eight weeks, six weeks, four weeks, two weeks or at least one week and any suitable time frame in between. Particulary preferred intervals depend on the study protocol. According to the general practice the most preferred range for such intervals are intervals of 4 to 12 weeks.

In a further preferred embodiment of the methods of the invention the composition to be administered to the subject/patient is the composition of the present invention.

A further embodiment of the invention relates to the use a pharmaceutically acceptable excipient (carrier, additive, capsule or diluent) enriched with at least one type of a stable rare isotope for the formulation of a composition of the invention.

The invention also relates to the use of a composition of the invention for monitoring of the compliance with an administration plan for the composition.

In a further alternative embodiment the invention relates to the use of a compound for the preparation of a pharmaceutical composition for the prevention, treatment or amelioration of a gastrointestina disease, a dermatological disease, a urological disease, a ophthalmological disease, a ear, nose and throat disease (otorhinolaryngological disease), a metabolic disorder, medical and surgical disorders which require medication, a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases, wherein said pharmaceutical composition comprises a pharmaceutically acceptable excipient (carrier, additive, capsule or diluent) enriched with at least one type of a stable rare isotope.

The figures show:

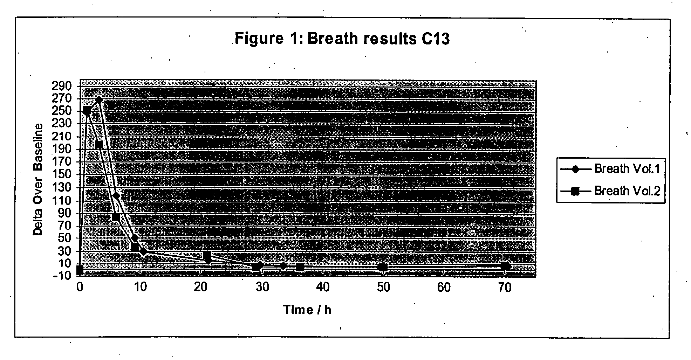

**Fig. 1: Breath test results:**
The figure shows the result of a breath test after the administration of $^{13}C_6$-glucose (1 g, two subjects). The relative content of $^{13}C$ (in units of DOB = delta over baseline) rises over a period of ca. 1 - 3 hours, after which it falls again, and returns to baseline values at around 36 - 48 hours.

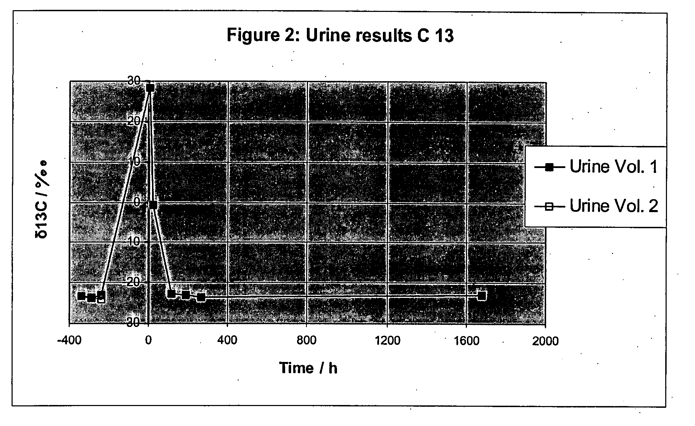

**Fig. 2: Urine test results:**
The figure shows the result of a urine test after the administration of $^{13}C_6$-glucose (1 g, two subjects). The relative amount of $^{13}C$ in urine shows a steep rise shortly after intake of $^{13}C_6$-glucose, and a drop to normal levels within approximately 5 days (only urine of subject 1 has been analysed).

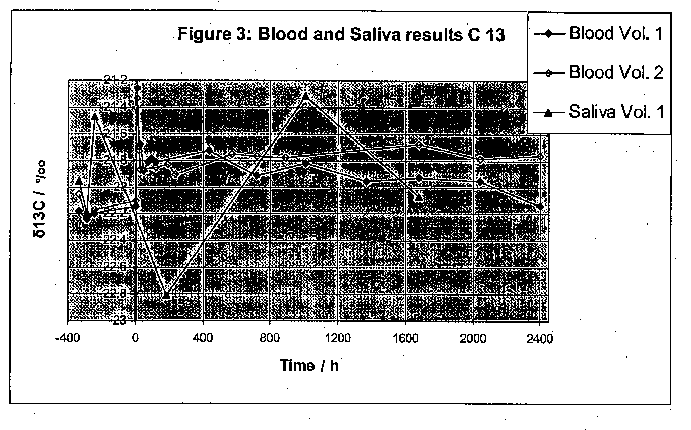

**Fig. 3: Saliva and blood test results**

The figure shows the result of a salvia and a blood test after the administration of $^{13}C_6$-glucose (1 g, two subjects). The analysed saliva samples show a relatively large variation, and no significant effect size. Blood samples of both volunteers show a marked increase in $^{13}C$-content at approximately 6 hours after ingestion, and a rapid decrease until about 50 hours after ingestion. The remaining effect size is small, and disturbed by large variations.

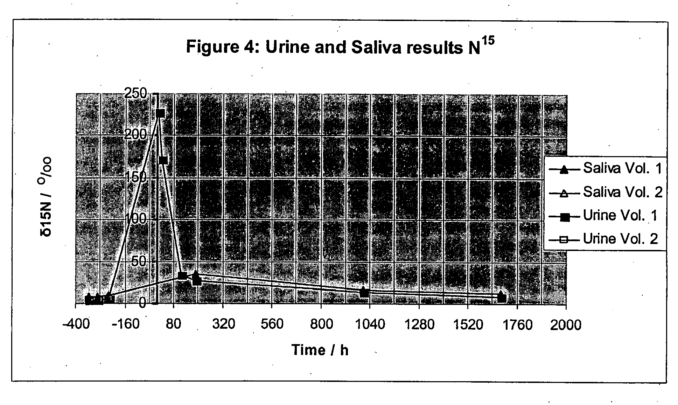

**Fig. 4: Saliva and blood test results**

The figure shows the result of a salvia and a blood test after the administration of $^{15}N$-L-leucine (1 g, two subjects). δ-$^{15}N$-values of urine show a steep increase after ingestion of the labeled substance, and a sharp drop. After about 5 days, the figure slowly returns to baseline values.

After a period of roughly 1 week, the δ-values in saliva are similar to urine-values, showing a slow decrease over time.

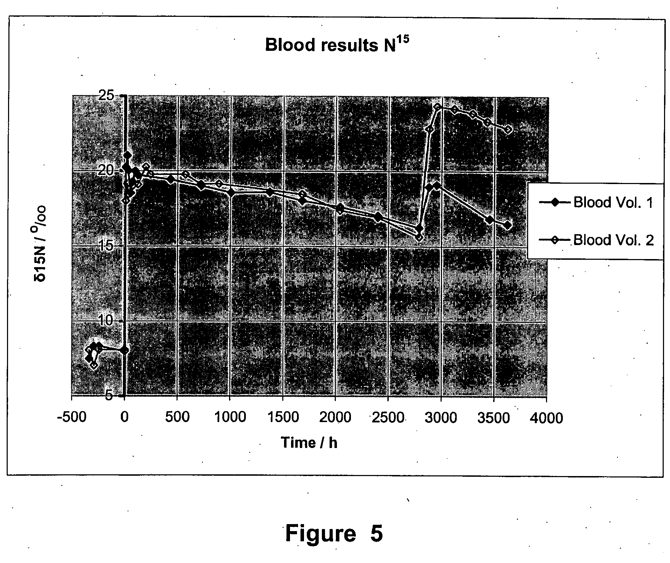

**Fig. 5: Blood test results**

The figure shows the result of a blood test after the administration of $^{15}N_3$-L-arginine (250 mg, one subject), respectively $^{15}N_4$-L-histidine (250 mg, one subject). The blood-δ-values show a fast increase in the first hours after ingestion, and then decrease slowly, with an approximate half-time of 8 000 — 10 000 h. It can be seen that the effect size is well above baseline fluctuations, and that the data for both subjects are very similar.

In a second experiment, 250 mg of $^{15}N_4$-L-arginine was ingested by one subject, and $^{15}N_3$-L-histidine by the other subject, 116 days after the first ingestion. The results are included in figure 5.

The invention is now described by reference to the following examples which are merely illustrative and are not to be construed as a limitation of scope of the present invention.

**Example 1:**

A small amount of a substance (e.g., an amino acid), labelled with a rare, stable isotope (e.g., $^{15}N$), is mixed with the medication to be taken. The same amount of indicator substance (several mg) should ideally be included in every pill/capsule of medication. A baseline blood sample is drawn from the patient before the intake of the first labelled pill. The patient then takes home a container with the labelled medication, to be taken according to a fixed schedule (e.g., once daily). After a certain period of time, the patient goes to the physician for a regular follow-up visit. Another blood sample will be taken.

Both blood samples (baseline and follow-up) will be sent to a laboratory for analysis (e.g., by mass spectrometry).

Here, the relative content of $^{15}N$ and $^{14}N$ in the blood will be measured. With the results of these measurements, and pharmacokinetic data to be obtained in a clinical trial, the amount of labelled substance ingested by the patient can be calculated, and thereby also the number of pills/capsules of medication. The compliance of each patient can thus be measured, and appropriate conclusions can be drawn.

Results:

As a first step to test the feasibility of this approach, a self-experiment was conducted by the inventors. The aim was to obtain first data on the effect size, the pharmacokinetic data in various body fluids, and the variability between subjects. Single, large doses of the following substances were ingested by the subjects:

$^{13}C_6$-glucose (1 g, two subjects)

$^{15}N$-L-leucine (1 g, two subjects)

$^{15}N_3$-L-arginine (250 mg, one subject)

$^{15}N_4$-L-histidine (250 mg, one subject)

Baseline and follow-up samples were taken from the following body fluids: blood, saliva, and urine. These samples were dried, milled, burned, and analysed by mass spectrometry. The results are given as δ-values, which are defined as follows:

$$\delta\text{-}^{15}N\ [^{13}C] = (r_{sample}\text{-}r_{standard})/r_{standard},$$

where $r_{sample}$ is the ratio of $^{15}N$ [$^{13}C$] over $^{14}N$ [$^{13}C$] in the sample, and $r_{standard}$ is the ratio of $^{15}N$ [$^{13}C$] over $^{14}N$ [$^{13}C$] in a standard.

Samples of exhaled breath were taken until 3 days after ingestion and analysed by IR-spectroscopy for $^{13}C/^{12}C$-ratios.

Glucose and leucine were ingested on day zero, while arginine and histidine were ingested on day 116.

$^{13}C$-results:

Breath test results (see figure 1): The relative content of $^{13}C$ (in units of DOB = delta over baseline) rises over a period of ca. 1 - 3 hours, after which it falls again, and returns to baseline values at around 36 - 48 hours.

Due to the small effect size and large variation in measured values of $^{13}C$-content, the usage of $^{13}C$-labeled glucose seems to be especially suitable for those cases, where the addition of amino acids is not desired, e.g., due to interactions with the pharmaceutical compound, or where glucose forms part of the standard formulation of the compound. Measurements of $^{13}CO_2$ in expired breath are only sensitive over 1 - 2 days, which is too short to be of practical use for this application.

In contrast, the results of measurements of $^{15}N$-content in blood show a large effect size, little variation over time, and also little differences between the two subjects. The slow decrease (= long biological half-life) is of great advantage when measuring compliance over a long time period (several weeks).

**Example 2: Sample preparation**

Blood: Appr. 4 ml of venous blood were drawn and given into a thoroughly cleaned glass vial. The sample was frozen immediately at -18°C and stored at this temperature.
Saliva: Saliva samples were obtained by letting the volunteers chew on "salivettes", i. e., cotton swabs, which were then centrifuged to extract saliva. The saliva samples were frozen at -18°C and stored at this temperature.
Urine: Urine was given into glass vials and frozen and stored at -18°C.
All samples were freezedried *in vacuo* at -70°C, and an appropriate amount of the resulting powder was given into Supremax glass tubes, together with an appropriate amount of copper oxide, evacuated, and sealed. The samples were then burned at 640 °C for 24 hours, to obtain $CO*2$ and $N2$, respectively.

**Example 3: Measurement of isotopic composition**

General description

Gas of the sample tubes was separated by freezing at -196°C ($N_2$) and - 78°C($CO_2$), and let into a mass spectrometer (Finnigan Delta +XL) according to manufacturer's specifications. The isotopic composition was measured and compared to a standard. Results are given as δ-values, which are defined as follows:

$$\delta\text{-}^{15}N\ [^{13}C] = (r_{sample}\text{-}r_{standard})/r_{standard},$$

where $r_{sample}$ is the ratio of $^{15}N$ [$^{13}C$] over $^{14}N$ [$^{13}C$] in the sample, and $r_{standard}$ is the ratio of $^{15}N$ [$^{13}C$] over $^{14}N$ [$^{13}C$] in a standard.
Samples of exhaled breath were taken until 3 days after ingestion and analysed by IR-spectroscopy for $^{13}C/^{12}C$-ratios.
Glucose and leucine were ingested on day zero, while arginine and histidine were ingested on day 116.

**References**

Avogaro A. et al., Diabetes 52(3), 759-802 (2003)
De Hoffmann E. and Stroobant V., Mass Spectrometry: Principles and Applications; John Wiley & Sons; 2nd edition (October 3, 2001)
Del Boca F. et al., Alcohol Clin Exp Res 20 (8), 1412-1417 (1996)
Ellard G. A. et al., Br J clin Pharmac 10, 369-381 (1980)

Korach-André M. et al., J Appl Physiol 93, 499-504 (2002)
Krumbiegel P. et al., Pediatr Gastroenterol Nutr 7(3), 333-40 (1988)
Lembcke B., Schweiz Rundsch Med Prax. 86(25-26): 1060-7 (1997)
Romagnuolo J., J Gastroenterol. May;97(5):1113-26 (2002)
Romijn J. A. et al., J Appl Physiol 88, 1707-1714 (2000)
Sidossis L. S. et al., J Clin Invest 95, 278-284 (1995)
Urquhart J., Clin Pharmacokinet 27(3), 202-215 (1994)
Woerle H. J. et al., Am J Pysiol Endocrinol Metab 284, E716-E725 (2003)
Wolfe R. R. et al., J Appl Physiol: Respirat Environ Exercise Pysiol 56(1), 221-229 (1984)
Wutzke K. D. et al., Monatsschr Kinderheilk 133, 291-295 (1985)

**Claims**

1. A composition comprising an agent for the prevention, alleviation, treatment or diagnosis of a disease and a pharmaceutically acceptable excipient, wherein said excipient is enriched with at least one type of a stable rare isotopes as a indicator for compliance with an administration plan for the composition.

2. The composition according to claim 1, wherein the pharmaceutically acceptable excipient is selected from the group of saccharides, gelatin, creatine, amino acids, (poly)peptides, proteins, lipids, fatty acids, and inorganic or organic salts.

3. The composition according to claim 1 or 2, wherein the at least one type of stable rare isotope is a $^{13}C$ or $^{15}N$ isotope.

4. The composition according of claim 2 or 3, wherein at least one amino acid or salt thereof is enriched with at least one type of stable rare isotopes.

5. The composition according to claim 4, wherein the at least one amino acid is selected from the group of leucine, histidine, arginine, isoleucine, lysine, methionine, phenylalanine, threonine, tryptophan, valine, glycin, alanin, asparagines, glutamine, cystein and serine.

6. The composition according of claim 2 or 3, wherein the saccharide is glucose, fructose, lactose, saccharose, hypromellose and/or starch.

7. An in vitro or ex vivo method for determination of the compliance with an administration plan for a composition, the method comprising the step of determining the amount of at least one type of a stable rare isotope in a sample, wherein said at least one type of stable rare isotope was enriched in a pharmaceutically acceptable excipient comprised in said composition.

8. A method for determination of the compliance with an administration plan for a composition, the method comprising the step of

(a) administering to a subject a composition comprising an agent for the prevention, alleviation, treatment or diagnosis of a disease and a pharmaceutically acceptable excipient enriched with at least one type of a stable rare isotope as a indicator for compliance;
(b) taking a sample from said subject; and
(c) determining the amount of said stable rare isotope in the sample.

9. The method according to claim 7 or 8, wherein said sample is a sample of a body fluid.

10. The method according to claim 9, wherein said body fluid is blood, serum, saliva, urine or sweat.

11. The method according to anyone of claims 7 to 10, wherein the amount of the stable rare isotope in the sample is determined by mass spectrometry.

12. The method according to anyone of claims 7 to 11, wherein the amount of the stable rare isotope is determined in a series of samples taken from a subject wherein the interval between the collection of the samples is 12 months, 9 months, 6 months, 16 weeks, 12 weeks, 10 weeks, 8 weeks, 6 weeks, 4 weeks, 2 weeks or 1 week.

13. The method according to anyone of claims 7 to 12, wherein said composition is a composition according to anyone of claims 1 to 7.

14. Use of a pharmaceutically acceptable excipient enriched with at least one type of a stable rare isotope for the formulation of a composition according to anyone of claims 1 to 6.

15. Use of a composition according to anyone of claims 1 to 7 for monitoring of the compliance with an administration plan for the composition.

16. Use of a compound for the preparation of a pharmaceutical composition for the prevention, treatment or amelioration of a gastrointestinal disease, a dermatological disease, a urological disease, a ophthalmological disease, a ear, nose and throat disease (otorhinolaryngological disease), a metabolic disorder, medical and surgical disorders which require medication, a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases, wherein said pharmaceutical composition comprises a pharmaceutically acceptable excipient enriched with at least one type of a stable rare isotope.

Figure 1: Breath results C13

Figure 2: Urine results C 13

Figure 3: Blood and Saliva results C 13

Figure 4: Urine and Saliva results N[15]

EP 1 637 163 A1

Figure 5

**European Patent Office**

**EUROPEAN SEARCH REPORT**

Application Number

EP 04 02 2450

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GROMAN ERNEST V ET AL: "Method to quantify tail vein injection technique in small animals." CONTEMPORARY TOPICS IN LABORATORY ANIMAL SCIENCE, vol. 43, no. 1, January 2004 (2004-01), pages 35-38, XP001204282 ISSN: 1060-0558 * the whole document * | 1-3, 7-11,13, 14,16 | A61K47/00 G01N33/48 |
| A | US 6 136 801 A (KELL MICHAEL) 24 October 2000 (2000-10-24) abstract, col. 6, line 47 - col. 7, line 66, claims 1 - 4 | 1-16 | |
| A | WO 01/34024 A (UNIV FLORIDA) 17 May 2001 (2001-05-17) abstract, claims 1 - 30 | 1-16 | |
| A | US 2003/194374 A1 (GRIGGS ROGER D ET AL) 16 October 2003 (2003-10-16) abstract, paragraphs [0026] - [0039] | 1-16 | TECHNICAL FIELDS SEARCHED (Int.Cl.7) |
| A | URQUHART JOHN ET AL: "Contending paradigms for the interpretation of data on patient compliance with therapeutic drug regimens" STATISTICS IN MEDICINE, vol. 17, no. 3, 15 February 1998 (1998-02-15), pages 251-267, XP009042297 ISSN: 0277-6715 p. 208/209, item 7.2. "Low Dose Chemical Markers" | 1-16 | A61K G01N |

The present search report has been drawn up for all claims

| Place of search | Date of completion of the search | Examiner |
|---|---|---|
| Munich | 26 January 2005 | Hoesel, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 04 02 2450

This annex lists the patent family members relating to the patent documents cited in the above-mentioned European search report.
The members are as contained in the European Patent Office EDP file on
The European Patent Office is in no way liable for these particulars which are merely given for the purpose of information.

26-01-2005

| Patent document cited in search report | | Publication date | Patent family member(s) | | Publication date |
|---|---|---|---|---|---|
| US 6136801 | A | 24-10-2000 | NONE | | |
| WO 0134024 | A | 17-05-2001 | AU | 777817 B2 | 04-11-2004 |
| | | | AU | 1589301 A | 06-06-2001 |
| | | | CA | 2390261 A1 | 17-05-2002 |
| | | | EP | 1227753 A1 | 07-08-2002 |
| | | | JP | 2003513694 T | 15-04-2003 |
| | | | MX | PA02004618 A | 02-09-2002 |
| | | | NZ | 518740 A | 30-04-2004 |
| | | | WO | 0134024 A1 | 17-05-2001 |
| | | | US | 2004081587 A1 | 29-04-2004 |
| US 2003194374 | A1 | 16-10-2003 | US | 2002095072 A1 | 18-07-2002 |
| | | | CA | 2461281 A1 | 26-09-2004 |
| | | | EP | 1462118 A1 | 29-09-2004 |
| | | | JP | 2004292450 A | 21-10-2004 |
| | | | CA | 2434029 A1 | 25-07-2002 |
| | | | EP | 1353703 A2 | 22-10-2003 |
| | | | WO | 02056919 A2 | 25-07-2002 |

EPO FORM P0459

For more details about this annex : see Official Journal of the European Patent Office, No. 12/82